# EUROPÄISCHE PATENTANMELDUNG

(11) **EP 4 258 812 A1**
(43) Veröffentlichungstag der Anmeldung: **11.10.2023**
(21) Anmeldenummer: 22166426.1
(22) Anmeldetag: 04.04.2022
(51) Int. Cl.: H05B 3/34, H02J 50/10, H02J 50/12, A47G 9/02, A47C 21/04, A47G 9/10, A61F 7/00

(54) **MOBILES, KABELLOSES HEIZSYSTEM MIT INDUKTIVER LADEMOEGLICHKEIT UND VERBESSERER GEBRAUCHSTAUGLICHKEIT**

(71) Anmelder: MOONICH Produktkonzepte und -realisierung GmbH, 82054 Sauerlach (DE)
(72) Erfinder: Keussen, Lars, 80469 München (DE)

(57) **Zusammenfassung**

Es wird gezeigt, wie etablierte Technologien aus dem Bereich der kabellosen Energieübertragung, der Akku-Technologie, und verschiedener Techniken von Heizgeräten zum Aufwärmen von Körperteilen, durch eine geschickte Verbindung mit Sensoren zur Präsenzerfassung dazu verwendet werden können, körpernahe, sichere, mobile Heizsysteme zu bauen, die in der Gebrauchstauglichkeit und im nachhaltigen ressourcenschonenden Umgang mit der Umwelt dem Stand der Technik überlegen sind.

## Beschreibung

### Technisches Gebiet

Kabellose Batterieladung, mobile Heizelemente, Heizkissen, Heizdecken, beheizte Kleidungsstücke, (medizinische) lokale Wärmebehandlung, Sitzheizung, Heizdecke, (zum Beispiel EP16154661A·2016-02-08)

### Stand der Technik

Induktive Ladesysteme für das kabellose Laden sind aus zahlreichen Einsatzgebieten in kommerziellen und professionellen Einsatzgebieten bekannt. Diese umfassen zum Beispiel elektrische Zahnbürsten, Herzschrittmacher, Smartphones oder ganze Elektrofahrzeuge. Im elektrischen Leistungsbereich zwischen 0,5 und 50W haben sich in den letzten Jahren Standards wie der Qi Standard des WPC (Wireless Power Consortium) oder das sogenannte "AirFuel" Systeme etabliert. Diese stellen eine Technologie zu Verfügung, um kabellos auf geringe Distanz (einige Millimeter bis Zentimeter) Energie von einem Sender auf eine, in der Regel kabellose, mobile Einheit mit einem elektrischen Energiespeicher (Batterie, Akku, Kondensator) zu übertragen. Die vorliegende Erfindung nutzt eine oder mehrere dieser Technologien.

Elektrische Sitzheizungen sind in zahlreichen Varianten insbesondere im Automobilbereich, bei Motorrädern oder auch im Wellnessbereich lange bekannt. In der Regel werden diese über eine Verkabelung mit einer zentralen Stromversorgung gespeist, oder mittels einer Batterie oder einer "Power Bank" mit elektrischer Energie versorgt. Entgegen Systemen, die die Heizleistung durch chemische Reaktionen, Verbrennung oder das Bereitstellen von Wärme durch extern vorgeheizte Stoffe gewährleisten, haben elektrische Systeme den Vorteil, dass sie ohne große Verluste elektrische Energie zu einem beliebigen Zeitpunkt in Wärme umwandeln können und die Menge der Energieumwandlung einfach kontrolliert werden kann. Die Verwendung von Akkumulatoren als wieder aufladbare elektrische Energiespeicher ist hier eine besonders geeignete und gängige Ausprägung. Die vorliegende Erfindung nutzt Akkumulatoren, in besonders vorteilhafter Ausprägung solche mit einer sehr hohen Ladezyklen-Zahl.

Aus der Gastronomie, bei Open Air Veranstaltungen, in Sportarenen oder auch in kirchlichen Gebäuden sind Heizstrahler-Systeme bekannt, die mittels erheblichem Energieaufwand eine beheizte Umgebung schaffen, um den Aufenthalt von Menschen, meist in Ansammlungen von mehreren Dutzend bei erschwerten Witterungsbedingungen möglichst angenehm zu gestalten. Solche Systeme "verschwenden" in der Regel einen erheblichen Anteil der Heizleistung an die Umwelt, da vergleichsweise hohe Temperaturen nötig sind, um aus einem gewissen Abstand durch Strahlung (infrarot) Wärme und Konvektion zur Verfügung zu stellen. Größere Mengen warmer Luft (Konfektion) entweichen oft in große Räume oder ins Freie und sind für das Wärmen der Personen damit nicht nutzbar. Eine individuelle Anpassung der Wärme an das persönlichen Wärmeempfinden in einer Gruppe von Menschen ist praktisch nicht möglich. Auch die Heizrichtung und der Abstand der Personen zur Wärmequelle sind solchen Systemen nicht optimal einstellbar. Die vorliegende Erfindung bezieht sich deshalb nicht auf solche Systeme, sondern auf individuelle, "persönliche" und mobile Heizgeräte, die körpernah eingesetzt werden können und vom Benutzer selbst auf sein Wohlbefinden eingestellt werden.

### Kurzbeschreibung der Erfindung

Es wird ein mobiles kabelloses System beschrieben, das es in besonders geeigneter Weise ermöglicht, elektrische Energie zu speichern und Wärme körpernah an einen Menschen oder eine Körperpartie abzugeben, ohne dabei erhebliche Energie an die Umwelt abzugeben.

Dieses System ist dadurch gekennzeichnet, dass es völlig kabellos in der Anwendung und auch beim Laden des elektrischen Akkumulators ist.

Außerdem ist es dadurch gekennzeichnet, dass eine Sensorik automatisch den Belegungszustand des Systems erkennen kann, und damit effektiv verhindert wird, dass die Energiequelle unnötig entladen wird, wenn keine Heizleistung gewünscht ist bzw. sich die Person vom Heizsystem entfernt hat.

Weiterhin verfügt das System über eine Sensorik, die es erlaubt, eine Temperatur nahe der Körpertemperatur einzustellen und zu halten, wobei der Benutzer die gewünschte Heizstufe nach seinem Empfinden/Bedarf einfach intuitiv wählen kann.

Zum Wiederaufladen des Systems wird das mobile Heizgerät (in der Regel zusammen mit einer Mehrzahl anderer mobiler Heizgeräte) kabellos in eine dafür vorgesehene Verwahrungsstation gelegt, die automatisch eine kabellose (induktive) Ladung des mobilen Energiespeichers übernimmt.

### Technische Aufgabe

Im Außenbereich der Gastronomie, bei Open-Air-Veranstaltungen und Konzerten, in Sportarenen, im Bereich Wellness und im kirchlichen Bereich ist der Erfolg der Veranstaltungen häufig von Wetterbedingungen beeinflusst oder muss sogar prinzipiell bei kühler Witterung stattfinden. Das Wohlfühlen der Gäste im Außenbereich oder in unbeheizten großen Gebäuden wie Kirchen ist deshalb jahreszeitlich und auch tageszeitlich sehr begrenzt. Stellt ein Gastwirt oder ein Veranstalter seinen Gästen deshalb eine Möglichkeit einer individuellen Heizung zur Verfügung, kann sich das sehr positiv auf die Verweildauer und die Menge der Besucher auswirken. Jedoch sind an die Flexibilität und die einfache Handhabung eines solchen "personalisierten" mobilen Heizsystems hohe Anforderungen gestellt.

Zum einen muss gewährleistet sein, dass die Heizelemente möglichst flexibel und individuell vom Gast zum Beispiel in einer Open-Air-Arena, einer Kirchensitzbank, auf einer Zuschauer-Tribüne oder in einem Skilift mitgenommen werden kann. Eine kabellose Lösung ist deshalb zwingend erforderlich, da Kabelanschlüsse zur Energiezufuhr in der Infrastruktur typischerweise nicht vorhanden sind und die Mobilität stark einschränken.

Die Handhabung von größeren Mengen solcher Heizelemente (typisch mehrere Dutzend bis mehrere Tausend) verlangt eine radikal einfache Bedienung, die das Anschließen von Kabeln, das Austauschen von Akkus oder dergleichen verbietet, das gilt während der Heiz-Phase, aber vor allem auch zum Wiederaufladen des Akkus vor oder nach der Benutzung. Das Personal des Gastronomen hat hier weder Zeit noch technische Kompetenz, um sich um das Aufladen zu bemühen.

Um den Gedanken des Umweltschutzes in außergewöhnlich hohem Maße gerecht zu werden, muss ein solches Heizsystem mehrere tausend Ladezyklen erlauben. In vielen Fällen versagen Akkus schon nach wenigen hundert Ladezyklen und stellen letztlich Batterie-Schrott dar, der entsorgt werden muss. Gängige Lithium-Ionen oder Lithium Polymer Akkus haben in der Regel nur einen relativ geringe Anzahl von Ladezyklen im Bereich von 500 bis max. 1000. Was konkret bedeutet, dass nach 500maligem Laden die Kapazität des elektrischen Energiespeichers nur noch ein Bruchteil der ursprünglichen Kapazität beträgt und damit die erwartete Leistung nicht mehr gegeben ist. Außerdem zeigt sich diese Akku-Technologie nachteilig bei der Anwendung in kälterer Umgebung, insbesondere bei Temperaturen unter 0°C, was aber gerade bei der Zielanwendung sehr nachteilig ist.

Ein konkretes Problem in vielen Anwendungsgebieten dieser Produkte ist es, dass der Gast nach der Veranstaltung, dem Restaurant-Besuch oder der Messe vergisst, die Heizung auszuschalten und diese dann längere Zeit noch weiter heizt und damit die Batterie zu Lasten der Umwelt entleert wird. Ein wesentlicher Vorteil des energieschonenden körpernahen Heizens ist damit beeinträchtigt.

In vielen Fällen ist die Heizleistung vorwiegend im Dämmerungsbereich morgens oder abends erforderlich, während tagsüber ausreichende Sonneneinstrahlung bestehen kann.

Es gilt also hier eine besonders geeignete technologische Form und Ausprägung von mobilen Energiespeichern mit Heizfunktion zu gestalten, die es ermöglicht, eine sehr lange Betriebsdauer des Produktes, eine sehr lange Lebensdauer und einen minimalen Bedienungsaufwand zu gewährleisten, so dass das individuelle Heizsystem nahezu ausnahmslos Energie für die kontrollierte Erwärmung der gewünschten Körperpartien zur Verfügung stellen kann, nicht aber die Umwelt unnötig aufheizt.

### Technische Lösung

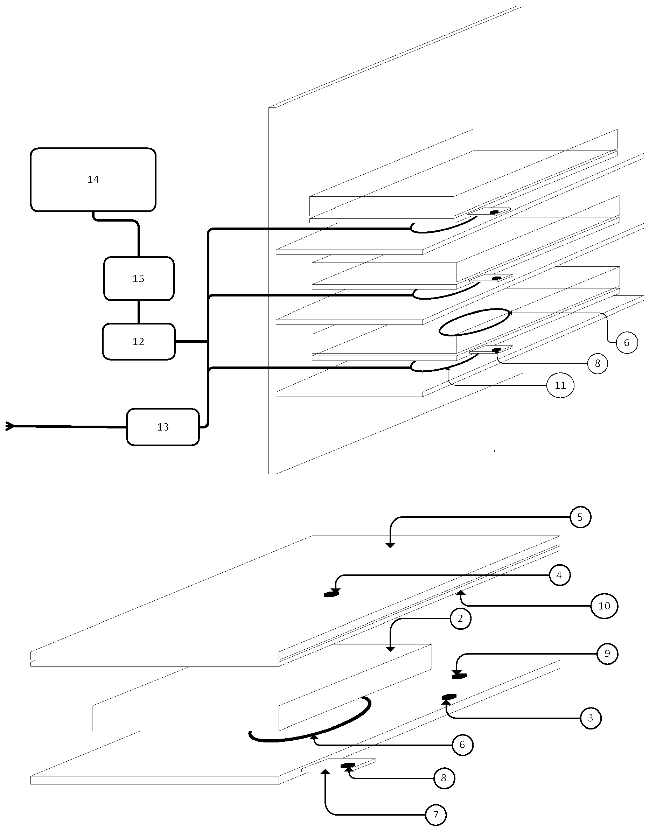

Die technische Lösung besteht aus einem System aus mehreren Heizgeräten (Fig 1) nebst dazugehörigen Ladestation(en) (Fig 2). In jedem Heizgerät sind wenigstens eine integrierte aufladbare Batterie (2) als elektrischer Energiespeicher, wenigstens ein Sensor zur Belegungserfassung (3), wenigstens ein Temperatursensor zur Temperaturerfassung (4), wenigstens eine elektrische Heizung (5) und wenigstens eine kabellose Energie-Empfängereinheit zum Laden der Batterie (6). Außerdem befindet sich (optional) am Heizgerät eine Bedieneinheit (7) zur Eingabe der Heizleistung und zur Darstellung des Betriebszustandes (8). Eine Steuer- und Regeleinheit (9) im Heizgerät verarbeitet die Sensor- und Eingabesignale und stellt den Betriebszustand des mobilen Heizgerätes dem Anwender in einfacher Weise dar, zum Beispiel durch eine LED Anzeige oder eine andere Art von Display. Desweiteren kann das Heizelement einen Reflektor enthalten (10), der die erzeugte Wärme gezielt in Richtung der Körpers abgibt und die Körperwärme gezielt reflektiert.

Die Ladestation (1) ermöglicht das Laden von mindestens einem, in der Regel aber einer Vielzahl von Heizgeräten, indem sie für jedes Heizgerät eine kabellose Energie Sendeeinheit zur Verfügung stellt (11). Diese wird beim Einlegen des mobilen Energiespeichers automatisch mit der Energie-Empfängereinheit (6) gekoppelt und damit das Laden der Batterie (2) ermöglicht, ohne dass ein Nutzer hierfür das System bedienen muss, ein Kabel einstecken muss oder die Batterie herausnehmen muss. Hierzu muss lediglich die Stromversorgung zu einem lokalen Energiespeicher (12) z.B. einem Akku, oder einer Netzversorgung (13) über ein Kabel in der Ladestation gegeben sein. Wahlweise kann der interne stationäre Energiespeicher auch über erneuerbare Energien wie z.B. ein Solarpanel (14), einen Windgenerator oder ein Wasserrad betrieben und über einen Laderegler (15) z.B. tagsüber geladen werden.

Die Batterie (2) wird vorzugsweise in einer LiFePO4 Chemie ausgelegt. Diese Akku-Technologie erlaubt den Betreib bei Temperaturen deutlich unter 0°C und in der Regel mehr als 2000 Ladezyklen. Damit wird über die Lebensdauer des Produktes von mehr als 5 Jahren in der Regel kein Akku Ersatz notwendig und damit wichtige Ressourcen gespart. Weiterhin besteht bei der LiFePO4 (auch LFP) Technologie keine sogenannte "Thermal Runaway Condition" (Explosion und Feuer im Fehlerfall). Es ist dadurch sichergestellt, dass außerordentlich hohe Sicherheit besteht, die auch einen Einsatz in rauer Umgebung erlaubt.

Ein Sensorsystem im Heizgerät (3) erlaubt die sichere Erkennung eins Menschen (oder eines Tieres) in unmittelbarere Nähe des Heizelements, an den die Wärme abgegeben wird. Spricht dieses Sensor System nicht an, ist also kein Körper in unmittelbarer Nähe der Heizung, dann wird die Heizung automatisch (nach einer Verzögerung) vom Steuergerät ausgeschalten.

Ein Sensor im Heizelement (4) wird auch dazu verwendet, die gewünschte Temperatur an der Oberfläche des Heizkörpers einzustellen. Diese liegt in der Regel deutlich oberhalb der Umgebungstemperatur, jedoch maximal unwesentlich oberhalb der Körpertemperatur von 37°C eines Menschen z.B. im Bereich bis 45°C. Dadurch kann je nach gewählter Heizstufe ein schnelles Aufwärmen und ein angenehmes Temperaturempfinden beim Benutzer erzeugt werden. Der Sensor (4) kann als eine Informationsquelle für eine zuverlässige Erkennung der Belegung mitgenutzt werden.

Wesentlicher Bestandteil des Systems zur einfachen Handhabung der Ladung ist das Bereitstellen der Ladestation in einer Weise, dass der Benutzer selbst und/oder der Betreiber des Systems, einfach zugänglich alle mobilen Einheiten in der Ladestation einlegen und verwahren kann, und diese durch die Formgebung so gestaltet ist, dass sich das Energie-Übertragungssystem automatisch koppelt und die mobile Einheit geladen wird. Damit wird sowohl eine sehr bedienerfreundliche Ladefunktion ohne jegliches Anstecken von Kabeln, Drücken von Knöpfen usw. gegeben. Konsequenterweise erlaubt die Status-Anzeige (8) am mobilen Teil auch die Anzeige des Ladezustandes in einfacher Weise. Womit der Benutzer auch beim Entnehmen direkt sehen kann, ob ein mobiler Heizkörper einen ausreichenden Ladezustand aufweist.

### Vorteilhafte Wirkungen

Eine vorteilhafte Wirkung eines intelligenten Sensorsystems im Heizgerät ist die Tatsache, dass auf Bedienelemente der Heizung zur Gänze verzichtet werden kann. Das Heizgerät selbst kann feststellen, ob sich ein Körper in der Nähe befindet und kann entsprechend selbst eine vordefinierte Temperatur (z.B. 37°C) einregeln. Sobald sich der Körper entfernt, wird die Heizleistung unterbrochen, um Energie zu sparen und die Betriebszeit der mobilen Heizung zu verlängern. In der Regel zeigt sich, dass für eine zuverlässige Erkennung eine Kombination aus mehreren Sensordaten erforderlich ist. Dies kann zum Beispiel die Bewegung, die Lage, die kapazitive Nähe oder die Körpertemperatur sein, oder eine beliebige Kombination solcher Sensordaten.

Heizen direkt an der gewünschten Körperpartie verringert dramatisch den Energiebedarf und schont damit erheblich Ressourcen und die Umwelt. In der Anwendung ergeben sich zum Beispiel im Außen-Gastronomiebereich besonders vorteilhafte Ausprägungen, wenn über einen elektrischen Zwischenpuffer (12) und die Ladung der mobilen Heizkörper in der Ladestation tagsüber Sonnenenergie gesammelt und gespeichert werden kann, die dann in der Dämmerung und nachts zum Wärmen eingesetzt werden kann.

Der Wegfall von Kabeln und damit verbundenen Steckverbindern ermöglicht es nicht nur, die Gebrauchstauglichkeit wesentlich zu verbessern, sondern trägt auch dazu bei, die wartungsfreie Lebenszeit des Systems zu erhöhen, da Steckerbinder- und Kabelbrüche zu den häufigsten Fehlern in elektrotechnischen System gehören.

Der Wegfall von Steckverbindern am mobilen Heizgerät erlaubt eine einfache komplette Kapselung der Elektronik und der Batterie. Damit ist das Eindringen von Flüssigkeiten, Staub und Schmutz technisch einfach vermeidbar. Ein Vorteil der gerade im beschrieben Anwendungsszenario eine wichtige Rolle für die Zuverlässigkeit spielt.

Die Auslegung der Ladestation als fest angeschlossenes Möbel, zum Beispiel im Eingangs- oder Durchgangsbereich von Veranstaltungsbereichen, erlaubt die einfache Handhabung "im Vorbeigehen". Auch das einfache gezielte Wiedereinsammeln beim Verlassen einer Veranstaltung wird so optimiert, wobei die Heizgeräte unmittelbar wieder verstaut und im Ladebetrieb sind, ohne zwischengelagert werden zu müssen.

### Kurze Beschreibung der Zeichnungsfiguren

Figur 1 zeigt das schematisch das Gesamtsystem mit verschiedenen Energieversorgungsmöglichkeiten der Ladestation und den Energie-Übertragungsmodulen bestehend aus Sender und Empfänger zu den mobilen Energiespeichern.
Figur 2 zeigt die Bestandteile des mobilen Energiespeichers mit Heizelement und Sensorik.

### Beschreibung der Ausführungsarten

Eine Ausführungsart der Erfindung betrifft ein mobiles kabelloses Heizkissen, in dem alle Elemente des Heizkörpers fest verbaut sind, welches eine Aufbewahrung in einer Ladestation und das Entnehmen der Heizkissen einfach und schnell möglich macht. Ebenso ist das Koppeln mit der Energiequelle beim Einlegen einfach möglich, da die Formgebung des Kissens ideal auf die Ladestation abgestimmt ist und die elektrische Kopplung zur Aufladung automatisch erfolgt.

Eine weitere Ausführungsart ist eine Decke, die zum Laden an einem Hacken aufgehängt wird, der die Kopplung des Senders mit dem Empfänger der Energieübertragung einfach ermöglicht.

Eine weitere Ausprägung sind Abstellplätze für Schuhe, Überschuhe oder Ski-Schuhe, welche die Heizkörpereinheit im Schuhwerk bereits verbaut haben. Durch das Abstellen auf einer formgebundenen Unterlage findet eine automatische Kopplung zur Energieübertragung statt.

Eine Ausführungsart der Ladestation besteht aus einer Station mit Solar-, Wind- oder Wasserkraft-Generator, der die gewonnene elektrische Energie in einem Niedervolt Akkumulator System vorzugsweise bei 12V speichert, und diese Energie aus dem Akku dann ohne nennenswerte Umwandlungsverluste für die kontaktlose Ladung der Heizgeräte verwendet werden kann. Sobald ausreichend Energie zur Verfügung steht, werden einige oder alle mobilen Energiespeicher geladen.

### Liste der angegebenen Unterlagen

Im Folgenden wird eine Liste von Patenten und Standards referenziert, die eine Vorstufe der aktuellen Erfindung darstellen und als solche den anerkannten Stand der Technik darstellen.

Außerdem wird auf gängige Standards für die kabellose Energieübertagung verwiesen.

### Patentdokumente

DE10255441A·2002-11-28 Temperaturmessung mit Reflektor-Folie
DE10029237A·2000-06-14 Sensoren, selektives Einschalten der Sitzheizung
EP10157802A·2010-03-25 Kapazitive Sensoren in Sitzheizung
DE202019105010U·2019-09-11 Mobile Flächenheizung mit Akku
US201916423826A·2019-05-28 Schnurloses Heizkissen
DE202017104346U·2017-07-20 Beheizbarer Schuhsohle
DE202016004548U·2016-07-21 Druckbasiertes automatisches Einschalten (Haustiere)
EP16154661A·2016-02-08 Heizgerät mit Powerbank
DE102013109731A·2013-09-05 Heizgerät mit "geringer elektromagnetischer Abstrahlung"
DE102011121511A·2011-12-16 beheizbares "Körnerkissen"
ITMI20100549A·2010-03-31 Heizkissen mit "Phase Change Material" Nichtpatentliteratur
The Qi Wireless Power Transfer System, Power Class 0 Specification (Wireless Power Consortium) https://www.wirelesspowerconsortium.com/knowledge-base/specifications/ download-the-qi-specifications.html
https://airfuel.org/

## Patentansprüche

1. System zum Bereitstellen von mobilen Heizkörpern, insbesondere Heizkissen oder Heizdecken, wobei der Heizkörper einen wieder aufladbaren Energiespeicher, ein Heizelement und eine Regelungseinheit mit Sensorik aufweist, **dadurch gekennzeichnet, dass** der Heizkörper mobil einsetzbar ist und völlig kabellos betrieben und geladen werden kann. Die Bedienung durch einen Laien-Benutzer ist so ausgelegt, dass maximal ein Bedienelement erforderlich ist.

2. System nach Anspruch 1, **dadurch gekennzeichnet, dass** die Abschaltung des Heizkissens bei Nichtbenutzung durch eine Kombination aus Sensoren und Zustandserkennung automatisch erfolgt, insbesondere ohne die Einwirkung eines Benutzers. Das unbeabsichtigte Entladen des Energiespeichers kann damit zuverlässig verhindert werden.

3. System nach Anspruch 1 oder 2, **dadurch gekennzeichnet, dass** eine Belegungserkennung des Heizkissens aufgrund von Sensordaten und Zustandserkennung automatisch und zuverlässig erfolgen kann, und damit der Benutzer das Kissen nicht einschalten muss.

4. System nach einem der vorangegangenen Ansprüche, **dadurch gekennzeichnet, dass** das Heizkissen maximal einen Bedienknopf hat, mit dessen Hilfe die Heizleistung durch den Benutzer einfach verändert werden kann. Die gewählte Heizleistung wird dabei in bevorzugter Weise durch eine visuelle Anzeige dargestellt.

5. System nach Anspruch 4, **dadurch gekennzeichnet, dass** die Heizleistung exklusiv oder zusätzlich zur einer visuellen Anzeige durch einen Vibrator bei der Wahl der Heizstufe angezeigt wird. Dies ermöglicht die einfache "blinde" Bedienung des Heizkissens.

6. System nach einem der vorangegangenen Ansprüche, **dadurch gekennzeichnet, dass** das Heizkissen nicht die Heizleistung steuert, sondern abhängig von Sensorparametern und der gewählten Heizstufe die Oberflächentemperatur des Heizkörpers geregelt wird. Dadurch wird ein Überhitzen vermieden und eine notwendige Anpassung der Heizstufe durch den Benutzer aufgrund von veränderten Umgebungsbedingungen überflüssig.

7. System nach einem der vorangegangenen Ansprüche, **dadurch gekennzeichnet, dass** ein Aufladen des internen Energiespeichers vor dem Gebrauch erfolgt, und dabei entstehende Verlustleistung zum Vorheizen des Kissens gespeichert und genutzt werden kann.

8. System nach einem der vorangegangenen Ansprüche, **dadurch gekennzeichnet, dass** der elektrische wieder aufladbare Energiespeicher auf einer einzelnen Batterie-Zelle basiert, und damit Umwandlungsverluste oder Kapazitätseinbußen bei der Abgabe der gespeicherten Energie und beim Laden des Energiespeichers minimiert werden.

9. System nach Anspruch 8 oder einem der vorangegangenen Ansprüche, **dadurch gekennzeichnet, dass** der elektrische Energiespeicher so ausgelegt ist, dass die Anzahl der Ladezyklen die Zahl 500 erheblich übersteigt und damit ein Wechsel des Energiespeichers über eine Lebenszeit von mehreren Jahren nicht notwendig wird.

10. System nach einem der vorangegangenen Ansprüche, **dadurch gekennzeichnet, dass** für den Energiespeicher Zellen aus gebrauchten Batterien verwendet werden können, und damit kritische Ressourcen entscheidend eingespart werden können, ohne die Sicherheit des Heizkissens zu beeinträchtigen.

11. System nach einem der vorangegangenen Ansprüche, **dadurch gekennzeichnet, dass** eine Mehrzahl von Heizkörpern in einer Ladestation geladen werden kann, so dass die dazu zur Verfügung stehende elektrische Energie ideal so ausgenutzt werden kann, dass die Betriebsbereitschaft einer Mehrzahl von Heizkissen aufgrund eines laufend ermittelten Benutzungsprofils in kürzest möglicher Zeit gegeben ist. Dies ermöglicht eine effiziente Ladung mehrerer Heizkissen insbesondere die Ladung an einer Ladestation, die aus regenerativen Energiequellen wie Solarstrom oder Windkraft gespeist werden.

12. System nach einem der vorangegangenen Ansprüche, **dadurch gekennzeichnet, dass** sich eine solar-basierte Ladeinheit am Heizkissen selbst befindet, die in direkter Weise Sonnenenergie in thermische Energie umwandelt und / oder zwischenspeichern kann.

13. System nach einem der vorangegangenen Ansprüche, **dadurch gekennzeichnet, dass** die Benutzung des Heizkissens völlig ohne Benutzerinteraktion möglich ist und nur anhand der ermittelten Sensordaten erfolgt.

14. System nach einem der vorangegangenen Ansprüche, **dadurch gekennzeichnet, dass** die Benutzung des Heizkörpers über eine Funkschnittstelle von einem Smartphone gesteuert werden kann.

15. System nach einem der vorangegangenen Ansprüche, **dadurch gekennzeichnet, dass** das Entfernen eines Heizkissens aus einem Wirkungsfeld durch eine Sensorik erkannt werden kann und zu einem Alarm führt. Dies kann insbesondere zum Diebstahlschutz dienen.

16. System nach einem der vorangegangenen Ansprüche, **dadurch gekennzeichnet, dass** es sich nicht explizit um ein Heizkissen, sondern um eine mobile Heizung handelt, die auch in anderer Form zur körpernahen Anwendung ausgeprägt sein kann, insbesondere als Decke, Strumpf, Jacke, Schuh, Handschuh, Mütze oder Bandage.

17. System nach einem der vorangegangenen Ansprüche, **dadurch gekennzeichnet, dass** sich das System auch für den medizinischen Einsatz zur Regulierung der Temperatur von Körperteilen einsetzen lässt.

18. System nach einem der vorangegangenen Ansprüche, **dadurch gekennzeichnet, dass** sich das System auch im Bereich der Nutz,- Zucht,- und Kleintierhaltung anwenden lässt.

19. System nach einem der vorangegangenen Ansprüche, **dadurch gekennzeichnet, dass** sich eine Mehrzahl von Heizelementen an der mobilen wieder aufladbaren Energiequelle befinden, die auf unterschiedliche Temperaturen eingestellt oder geregelt werden können.
